Europäisches Patentamt

European Patent Office

Office européen des brevets

(19)

(11) EP 0 578 975 B2

(12) **NEUE EUROPÄISCHE PATENTSCHRIFT**

(45) Veröffentlichungstag und Bekanntmachung des Hinweises auf die Entscheidung über den Einspruch:
**12.01.2005 Patentblatt 2005/02**

(45) Hinweis auf die Patenterteilung:
**21.04.1999 Patentblatt 1999/16**

(21) Anmeldenummer: **93109438.7**

(22) Anmeldetag: **14.06.1993**

(51) Int Cl.⁷: $G01N\ 33/36$, $G01N\ 21/89$, $D01H\ 13/26$, $D03C\ 19/00$, $D04B\ 37/00$

(54) **Verfahren und Vorrichtung zur Beurteilung der Auswirkung von Garnfehlern auf Gewebe oder Gewirke**

Method and apparatus for predicting the effect of yarn defects on the appearance of textiles or fabrics

Procédé et appareil de prédiction de l'effet de défauts d'un fil sur l'apparence de tissus ou tricots

(84) Benannte Vertragsstaaten:
**BE DE ES FR GB IT**

(30) Priorität: **18.06.1992 CH 192692**

(43) Veröffentlichungstag der Anmeldung:
**19.01.1994 Patentblatt 1994/03**

(73) Patentinhaber: **ZELLWEGER LUWA AG**
**8610 Uster (CH)**

(72) Erfinder: **Hoeller, Robert, Dr.**
**CH-8610 Uster (CH)**

(74) Vertreter: **Ellenberger, Maurice et al**
**Zellweger Luwa AG**
**Wilstrasse 11**
**8610 Uster (CH)**

(56) Entgegenhaltungen:
**EP-A- 0 199 552          EP-A- 0 249 741**
**EP-A- 0 439 659**

• **Basic and Practice of the Evennesss Testing; April 1986, Keisokki Kogyo Co.,Osaka JP**

EP 0 578 975 B2

**Beschreibung**

Die vorliegende Erfindung betrifft ein Verfahren und eine Vorrichtung zur Beurteilung der Auswirkung von Garnfehlern auf aus dem betreffenden Garn hergestellte Gewebe oder Gewirke durch Simulation des Warenbildes.

Traditionelle Verfahren dieser Art verwenden zur Simulation des Warenbildes sogenannte Schautafeln, das sind trapezförmige oder rechteckige Kartons oder Bleche, die mit dem jeweiligen Garn spiralförmig umwickelt werden, wodurch eine Art von Quasigewebe oder -gewirke gebildet wird, aus dem eventuelle Fehlermuster gut erkennbar sind. Die Schautafeln sind somit ein wertvolles Hilfsmittel zur Abschätzung, ob und inwieweit ein bestimmtes Garn für eine bestimmte Ware geeignet ist, und sie ermöglichen Voraussagen über eines der wichtigsten Qualitätsmerkmale des Fertigprodukts nämlich dessen Aussehen.

Allerdings ist die Herstellung der Schautafeln durch Umwickeln von Blechen relativ arbeitsintensiv und wirkt auch nicht mehr zeitgemäss, so dass ein Bedürfnis nach einer neuen Methode zur Simulation des Warenbildes besteht. Zwar ist aus der Veröffenttlichung mit dem Titel: "Basic Practice of The Evenness Testing", April 1986, Keisokki Kogyo Co., Ltd. Osaka. JP ein Verfahren bekannt, mit dem simuliert werden kann, wie periodische und nichtperiodische Fehler im Garn sich auf Gewebe auswirken können, wenn bestimmte Voraussetzungen erfüllt sind. Dabei werden Muster, die in Geweben auftreten können, rechnerisch ermittelt, d.h. mit Formeln ausgehend von Eigenschaften des Garns berechnet. Dabei werden aber nur die Auswirkungen typischer, isoliert betrachteter Fehler in einem Garn dargestellt Nach diesem Verfahren erzeugte Figuren stellen somit Vereinfachungen dar, die durch die Theorie abgeleitet sind

Aus der EP 0 199 552 ein Verfahren zur rechnergestützten Simulation des Aussehens eines Gewebes bekannt. bei dem eine Garneigenschaft (schon vorher) bekannt oder beschafft sein muss. Die genannte Garneigenschaft z.B. Denier, Färbbarkeit usw. ist eine Eigenschaft, die sich auf ein ganzes und längeres Garnstück bezieht. Die einmal festgestellten Werte für diese Eigenschaft werden gemittelt, so dass ein Rechner nur mit einem einzigen Wert weiterrechnet. der die Eigenschaft des Garnes darstellt. Dieser Wert wird in Bildpunkte umgewandelt, die so aneinandergereiht werden, dass ein Bild eines Gewebes entsteht, aus dem entnommen werden kann, wie die Mischung von Garnen mit verschiedenen Eigenschaften oder die Verbindungen zwischen den Garnen sich optisch auswirken.

Aus des Promotionsschrift "Filament Blending in Air-Jet Texturing" (A.K.Seth) Oct, 1988, ist ein Verfahren zur Beurteilung von Mischgarnen bekannt, bei dem aufeinanderfolgende Garnabschnitte mittels einer Fernsehkamera erfasst, die gewonnenen Signale integriert und in Helligkeitswerte umgerechnet werden, aus welchen zur Simulation des Erscheinungsbildes eines aus dem untersuchten Garn hergestellten Gewebes ein Helligkeitsprofil des Garns erstellt wird.

Die genannte neue Methode sollte möglichst wenig Arbeitsaufwand erfordern, sie sollte flexibel sein und sie sollte zuverlässige und reproduzierbare Resultate liefern.

Diese Aufgabe wird durch die Merkmale der Patentansprüche 1 und 6 gelöst.

Mit der Erfindung werden also die Schautafeln auf elektronischem Weg hergestellt, wobei bei Verwendung eines Gleichmässigkeitsprüfers, wie beispielsweise des USTER TESTER (USTER-eingetragenes Warenzeichen der Zellweger Uster AG), als Messvorrichtung zur Untersuchung der genannten Parameter, die elektronischen Schautafeln aus den üblicherweise produzierten Daten berechnet werden. Mit dem USTER TESTER, der beispielsweise in der EP-A-0 249 741 und in der CH-A-671 105 beschrieben ist, wird unter anderem die Gleichmässigkeit und/oder die Haarigkeit einer Garnprobe untersucht und in Form eines Diagramms, eines Wellenlängenspektrums und anderer grafischer Darstellungen der Schwankungen der gemessenen Parameter auf einem Bildschirm und/oder einem Drucker dargestellt. Mit der Gleichmässigkeit und der Haarigkeit liegen zwei Parameter vor, die für das spätere Warenbild wesentlich sind und die mit einem relativ geringen Softwareaufwand zur Simulation der Schautafeln verarbeitet werden können

Im folgenden wird die Erfindung anhand eines Ausführungsbeispiels und der Zeichnungen näher erläutert:

| | |
|---|---|
| Fig 1 | eine Perspektivdarstellung einer Prüfanlage zur Bestimmung der Masseschwankungen eines textilen Prüfguts. |
| Fig. 2 | ein mit der Prüfanlage von Fig. 1 gewonnenes Diagramm einer Einzelprobe |
| Fig. 3 | einen Ausschnitt aus einer Sammelgrafik mit Spektrogrammen von zwei Proben; und |
| Fig.4a,b | Schautafel-Simulationen der beiden Proben von Fig. 3. |

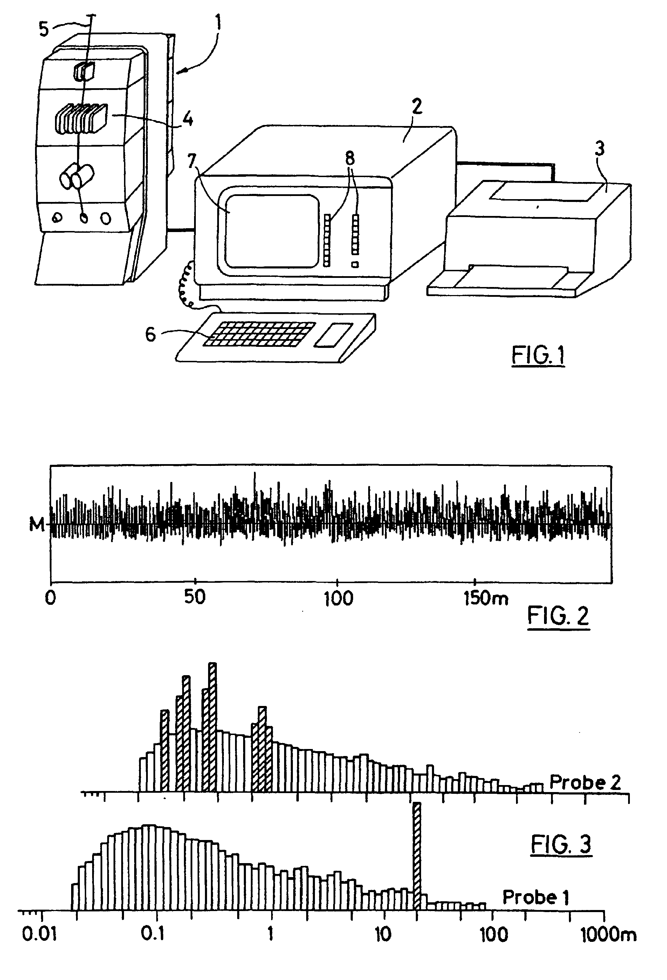

Die in Fig. 1 dargestellte Prüfanlage ist der USTER TESTER der Zellweger Uster AG, der zur Bestimmung von Material- und Qualitätskenngrössen von textilem Prüfgut, wie beispielsweise Garnen, verwendet wird. Diese Kenngrössen sind beispielsweise Masseschwankungen, Haarigkeit oder Struktur (Drehung) des untersuchten Garns (siehe dazu beispielsweise CH-A-671 105, EP-A-0 249 741, US-A-5 030 841), wobei sich Fehler dieser Kenngrössen in unerwünschter Weise auf das textile Fertigprodukt auswirken.

Die Prüfanlage besteht in bekannter Weise aus dem eigentlichen Prüfgerät 1, einer Auswerte- und Bedienungseinheit 2 und einem Drucker 3. Das Prüfgerät 1 ist mit einem oder mehreren Messmodulen 4 versehen, welche Messorgane für die zu untersuchenden Kenngrössen aufweisen. Das mit dem Bezugszeichen 5 bezeichnete Prüfgut oder Garn wird durch die Messorgane transportiert, die die Masse, die Haarigkeit und/oder die Struktur fortlaufend messen und in elektrische Signale umwandeln. Das geprüfte Garn 5 wird nach der Messung abgesaugt.

In der Auswerte- und Bedienungseinheit erfolgt die Signal- und Datenverarbeitung und die Funktionskontrolle der Prüfanlage. Ueber eine Tastatur 6, einen Bildschirm 7 und Steuertasten 8 werden Variable, Messbedingungen und die gewünschte Darstellung der Resultate eingegeben, und auf dem Bildschirm 7 erscheinen Messablauf und Resultate in numerischer und grafischer Form. Der Drucker 3 dient ebenfalls zur Ausgabe von Messwerten und von grafischen Darstellungen und insbesondere auch zur Ausgabe von vollständigen Prüfberichten.

Das unmittelbare Ergebnis der Untersuchung einer Garnprobe 5 mit der dargestellten Prüfanlage ist das in Fig. 2 dargestellte Diagramm, welches die Schwankungen der untersuchten Kenngrössen über die Länge des Prüfguts anzeigt. Wenn beispielsweise die Haarigkeit untersucht wird, dann ist diese als die totale Länge der vom Garnkörper abstehenden Fasern innerhalb einer bestimmten Messfeldlänge definiert. Die Haarigkeit eines Garns ist dann der über die gesamte Prüflänge gebildete Mittelwert.

Das Diagramm zeigt nun die Schwankungen der untersuchten Kenngrösse um einen Mittelwert M, der einem normierten Wert des den Garnquerschnitt repräsentierenden Signals entspricht. Aus diesen Schwankungen, die bei Untersuchung der Masseschwankungen in Prozenten und bei Messung der Haarigkeit in Absolutwerten angegeben sind, lässt sich die Streuung oder Standardabweichung errechnen.

Bestimmte Grenzen übersteigende Schwankungen sind ein Hinweis auf einen Fehler, wobei bei den Fehlern bekanntlich zwischen periodischen und nichtperiodischen Fehlern unterschieden wird. Periodische Fehler weisen definitionsgemäss eine bestimmte Wellenlänge auf und sind anhand des Wellenlängenspektrogramms einfach zu detektieren, wobei im Spektrogramm auftretende Kamine einen Fehler anzeigen. In Fig. 3 sind die Spektrogramme der Masseschwankungen von 2 Proben dargestellt; Probe 1 hat einen Kamin mit einer Wellenlänge von 20 m und Probe 2 hat vier Kamine, die im Bereich unterhalb von 50 cm liegen.

Je nach der Breite des späteren Gewebes oder Gewirkes und je nach der Wellenlänge des periodischen Fehlers treten im Fertigprodukt unerwünschte Muster auf, die das Fertigprodukt vielfach unbrauchbar machen. Es sei in diesem Zusammenhang auf die Publikation "Evenness Testing in Yarn Production: Part von R. Furter, The Textile Institute, 1982, verwiesen, in der auf Seite 60 ff der Einfluss von periodischen Masseschwankungen auf Gewebe und Gewirke erläutert wird. Diesen Erläuterungen ist unter anderem zu entnehmen, dass kurzperiodische Masseschwankungen mit einer Wellenlänge von 1 bis 50 cm zu einem sogenannten Moiré-Muster führen, und dass langperiodische Masseschwankungen mit einer Wellenlänge von über 5 m relativ starke Querstreifen im Fertigprodukt verursachen können. Entsprechend müsste ein Gewebe aus Garn von Probe 1 (Fig. 3) Querstreifen und ein solches aus Garn von Probe 2 ein Moiré-Muster aufweisen. Wenn nicht die Masseschwankungen sondern Haarigkeit oder Struktur untersucht werden, dann gelten grundsätzlich die gleichen Zusammenhänge, nur sind die Auswirkungen periodischer Fehler auf das Fertigprodukt bei der Haarigkeit eher stärker und bei der Struktur tendenziell eher geringer.

Nahezu periodische Fehler führen im Fertigprodukt zu einem unruhigen Aussehen, zu einem sogenannten "wolkigen Charakter" und von nichtperiodischen Fehlern, den sogenannten Imperfektionen, sind insbesondere die Nissen äusserst störend, weil sie in der Regel andere Reflexionseigenschaften aufweisen als fehlerfreies Garn und beispielsweise Farbe anders oder gar nicht annehmen. Die Imperfektionen werden beim USTER TESTER registriert und gezählt und getrennt nach Fehlerarten, Dickstellen, Dünnstellen und Nissen, angezeigt und/oder ausgedruckt.

Das Signal des Messorgans des Messmoduls 4 (Fig. 1), das im Diagramm von Fig. 2 wiedergegeben ist, und/oder das von der Auswerte- und Bedienungseinheit 2 verarbeitete Signal, beispielsweise das Spektrogramm von Fig. 3 oder die Anzahl der Nissen, wird nun dazu verwendet, auf dem Bildschirm 7 ein Abbild des aus dem untersuchten Garn hergestellten Gewebes oder Gewirkes zu erzeugen. Dieses Abbild zeigt dann der Bedienungsperson unmittelbar die Auswirkungen der vom USTER TESTER gefundenen Garnfehler auf das Fertigprodukt und ermöglicht somit eine Prognose des späteren Warenbildes.

Die Simulation des Warenbildes erfolgt dadurch, dass die genannten Signale, welche mit dem Volumen oder der Oberfläche des Garns zusammenhängende Parameter repräsentieren, in Grauwerte oder Farbwerte umgerechnet und einem oderer mehreren Bildpunkten (Pixeln) zugeordnet, und dass diese Pixel anschliessend auf dem Bildschirm 7 und gegebenenfalls auch auf dem Drucker 3 wiedergegeben werden. Die Parameter können in der Simulation alternativ oder beliebig kombiniert zur Anzeige gebracht werden. Für diese Wiedergabe ist die "Fadenführung" variabel, das heisst, man kann das Garn wie auf einer konventionellen Schautafel spiralförmig wickeln, wobei der Bildschirm das Warenbild auf der Vorderseite der Schautafel wiedergegeben würde, oder man kann gleichsam die Schautafel transparent machen und deren Vorder- oder Rückseite einander überlagern. Oder man kann das Garn jeweils nur in einer Richtung führen, beispielsweise von links nach rechts, so dass der Faden am rechten Ende der Tafel abgeschnitten und anschliessend an der linken Seite wieder angesetzt wird. Oder man kann das Garn kreuzweise überlagern, was einem Gewebe entspricht, oder man kann ein Gewirk nachbilden. Dabei kann die Bildauflösung beliebig gewählt werden; beispielsweise können mehrere nebeneinanderliegende Fäden zusammengefasst werden, wobei die Intensität der Bildpunkte dem Mittelwert dieser Fäden entsprechen würde. Es sind auch selektive Auswertungen der Daten möglich, indem beispielsweise aus dem Spektrogramm nur einzelne Kamine oder nur die Differenz zum Idealspektrogramm angezeigt werden.

Als Ausgangspunkt für die Berechnung der Grau- oder Farbwerte stehen grundsätzlich zwei Kategorien von Signalen zur Verfügung, und zwar einerseits das unmittelbar vom Messorgan erzeugte Signal entsprechend dem Diagramm von Fig. 2, und andererseits ein Signal, das bereits das Ergebnis einer vom USTER TESTER vorgenommenen Auswertung darstellt, also beispielsweise das Spektrogramm oder das Ergebnis einer Nissenzählung. Ein Signal dieser zweiten Kategorie würde also Mittelwerte oder Streuungen ausgesuchtertextiltechnisch relevanter Grössen darstellen. Aus beiden Signalkategorien wird dann ein Garnsignal für die Darstellung auf dem Bildschirm simuliert, wobei für die Simulation gewisse Charakteristika durch aus der Bildverarbeitung bekannte Massnahmen, wie Kontrastverstärkung, Einfärbung und dergleichen hervorgehoben werden können.

Für die Berechnung gilt allgemein:

$$I = F(y)$$

(I: Helligkeits- oder Farbstufe, y: Masse, Haarigkeit, Struktur; Abweichung vom Mittelwert)

Für die Berechnung aus der 1. Kategorie von Signalen, also aus dem Diagramm, gilt:

$$y = f(x)$$

(x: Position in Garnlängsrichtung)

wobei die y-Werte direkt dem Diagramm entnommen werden.

Für die Berechnung aus der 2. Kategorie von Signalen gilt für periodische Fehler (Spektrogramm):

$$y = \sum_{i=1}^{n} \left[ a_i \cdot \sin \left( \frac{\pi}{\lambda_i} \cdot x \right) \right]$$

(a: Amplitude der Wellenlänge
i: Index der Wellenlänge im Wellenlenlängenspektrogramm
$\lambda$: Wellenlänge
x: Position in Garnlängsrichtung)

Die y-Werte entsprechen also einem rekonstruierten Diagramm. Eventuelle Kamine im Spektrogramm werden entweder von Hand markiert oder mittels der in der CH-Patentanmeldung 2651/91 beschriebenen Methode detektiert. Alternativ zur Berechnung anhand der angegebenen Formel kann auch eine schnelle Fourier-Transformation (FFT) verwendet werden.

In der Praxis gilt für die Berechnung von I vorzugsweise:

$$y_1 = \sum_{i=1}^{n} \left[ a_i \cdot \left\{ 1 + \sin \left( \frac{\pi}{\lambda_i} \cdot x \right) \right\} \right]$$

($y_1$: Abweichung vom Minimalwert; dabei ist y so verschoben, dass $y_1$ immer positiv ist)

$$I = k - \sqrt{y_1} \quad \text{oder} \quad I = K.y_1$$

(k, K: konstanter Multiplikator)

Seltene zufällig auftretende Fehler (Imperfektionen) werden dem entsprechenden Kanal des USTER TESTER entnommen und ihrer Intensität und Häufigkeit entsprechend im Bild dargestellt, wobei der Ort der betreffenden Bildpunkte durch Zufallszahlen bestimmt wird.

Die Frage, ob man für die nicht-seltenen Fehler vom Diagramm ausgehen und auf dem Bildschirm das reale Garn abbilden, oder ob man vom Spektrogramm ausgehen und ein aus statistischen Werten rekonstruiertes, simuliertes Garn abbilden soll, wird heute zugunsten des Spektrogramms beantwortet werden. Denn im Spektrogramm werden in der Regel wesentlich kleinere Wellenlängen registriert als im Diagramm, da ein Diagramm hoher Auflösung eine sehr grosse Datenmenge ergeben würde. Somit steht oft kein Diagramm mit der zur Darstellung des Schaubildes erforderlichen Auflösung zur Verfügung.

Ausserdem ist fraglich, ob beispielsweise Nissen in diesem Abbild des realen Garns erkannt würden. Entnimmt man hingegen die Nissen dem Nissenkanal und streut sie mit entsprechender Dichte und unter geeigneter Kontrastverstärkung, wie zum Beispiel Einfärbung, in das Bild, dann werden sie mit Sicherheit auch auf dem Bildschirm erkannt. Hingegen verlangt die fortlaufende Rekonstruktion des Diagramms aus dem Spektrogramm einen wesentlich höheren Rechenaufwand als die direkte Umsetzung des Diagramms in Helligkeits- oder Farbwerte.

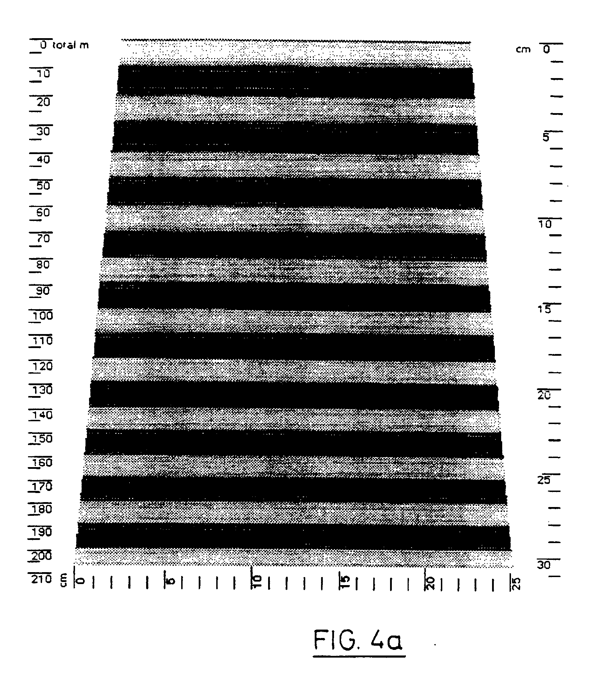

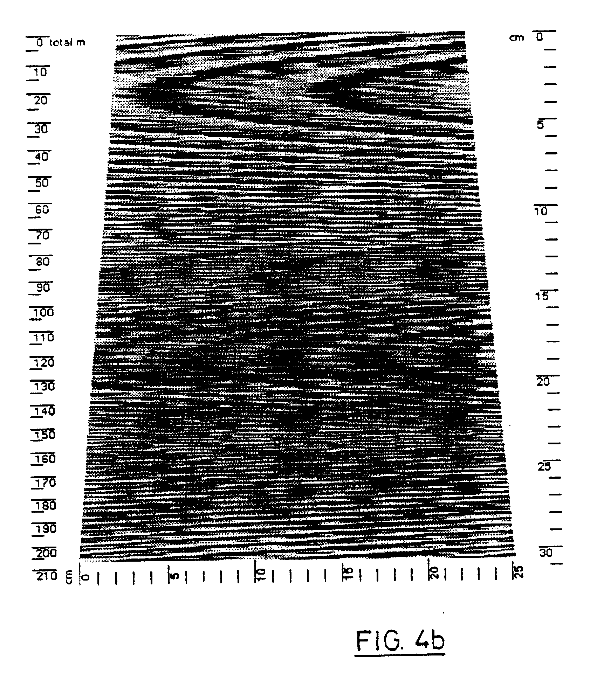

Die Fig. 4a und 4b zeigen je eine aus den Spektrogrammen von Fig. 3 berechnete Schautafel-Simulation für die beiden Proben 1 beziehungsweise 2. Diese Simulationen ergeben das für den Fachmann erwartete Ergebnis mit Querstreifen für Probe 1 und einem Moiré-Muster für Probe 2, welche durch die durch die schraffierten Kamine in den beiden Spektrogrammen gekennzeichneten periodischen Fehler bedingt sind. Dieses Ergebnis be weist die Praxistauglichkeit des beschriebenen Verfahrens.

## Patentansprüche

1. Verfahren zur Beurteilung der Auswirkungen von mit dem Volumen und/oder dem Durchmesser und/oder der Oberfläche zusammenhängenden Parametern eines Garnes auf Gewebe und Gewirke, die aus dem betreffenden Garn hergestellt sind, durch Simulation eines Warenbildes, **dadurch gekennzeichnet, dass** das Garn in seiner Längsrichtung durch ein Messorgan (4) hindurch bewegt'wird, das Signale erzeugt, die Schwankungen eines untersuchten Parameters über die Länge des Prüfguts anzeigen, dass die Signale in Grauwerte oder Farbwerte umgerechnet und diese Bildpunkten zugeordnet werden, die einer Position in Garnlängsrichtung entsprechen und dass diese Bildpunkte gemäss ihrer Position längs des Garns aneinandergereiht werden,

   dass die genannten Parameter durch Masse, Durchmesser, Haarigkeit und/oder Struktur des Garns (5) gebildet sind,

   dass die Umrechnung der genannten Parameter nach der Formel I = F (y) erfolgt, wobei I die Helligkeits- oder Farbstufe und y den betreffenden Parameter bezeichnet,

   dass aus den unmittelbar gemessenen Schwankungen der genannten Parameter ausgesuchte textiltechnisch relevante Grössen abgeleitet und diese abgeleiteten Grössen für die Umrechnung verwendet werden, wobei für die Wiedergabe der Bildpunkte aus diesen textiltechnisch relevanten Grössen ein Garnsignal simuliert wird und dass für die Simulation gewisse Charakteristika durch aus der Bildverarbeitung bekannte Massnahmen, wie Kontrastverstärkung, Einfärbung und dergleichen, hervorgehoben werden.

2. Verfahren nach Anspruch 1, **dadurch gekennzeichnet, dass** die unmittelbar gemessenen Schwankungen der genannten Parameter, die sogenannten Diagramme, ausgewertet und dass die Werte für die Umrechnung direkt den Diagrammen entnommen werden.

3. Verfahren nach Anspruch 1 **dadurch gekennzeichnet, dass** für die Umrechnung die seltenen F ehler im Garn (5) die sogenannten Imperfektionen, verwendet und dass diese mit einer der Intensität und Häufigkeit der Messdaten entsprechenden Intensität und Häufigkeit in dem Bild mit dem simulierten Garnsignal dargestellt werden.

4. Verfahren nach Anspruch 1, **dadurch gekennzeichnet, dass** für die Umrechnung das Wellenlängenspektrogramm der Schwankungen eines Parameters verwendet wird, wobei eine Untersuchung des Wellenlängenspektrogramms auf sogenannte Kamine, das sind merklich über die ideale Spektrogrammkurve hinausragende Werte, erfolgt.

5. Verfahren nach Anspruch 4, **dadurch gekennzeichnet, dass** die Umrechnung nach der Formel

$$y = \sum_{i=1}^{n} \left[ a_i \cdot \sin\left(\frac{\pi}{\lambda_i} \cdot x\right) \right]$$

erfolgt, wobei λ die Wellenlänge eines Kamins, a dessen Amplitude und i dessen Index im Wellenlängenspektrogramm, und x die Position des Kamins in Längsrichtung der Garnprobe (5) bezeichnet und y eine Rekonstruktion des betreffenden Parameters darstellt.

6. Vorrichtung zur Beurteilung der Auswirkungen von mit dem Volumen und/oder dem Durchmesser und/oder der Oberfläche zusammenhängenden Parametern eines Garns auf Gewebe und Gewirke, die aus dem betreffenden Garn hergestellt sind, durch Simulation eines Warenbildes, **gekennzeichnet durch**, ein Messorgan (4) zur Bestimmung von Signalen, die Schwankungen eines Parameters über die Länge des Garns entsprechen, **durch** einen Rechner (2) zur Umrechnung der Signale in Grauwerte oder Farbwerte, mit Mitteln zur Zuordnung der Grauwerte oder Farbwerte zu Bildpunkten, **durch** einen Bildschirm (7) und/oder einen Drukker (3) und **durch** Steuermittel zur Wiedergabe der Bildpunkte auf dem Bildschirm und/oder dem Drucker gemäss der Position in Gamlängsrichtung, zwecks Simulation eines aus dem untersuchten Garn hergestellten Gewebes oder Gewirkes, wobei die wiedergegebenen Bildpunkte eine Simulation des untersuchten Garns (5) darstellen, welche anhand von statistischen Untersuchungen textiltechnisch relevanter Grössen des bei der Prüfung der Garnprobe gewonnenen Garnsignals gewonnen wurden.

7. Vorrichtung nach Anspruch 6, **dadurch gekennzeichnet, dass** die wiedergegebenen Bildpunkte ein rekonstruiertes Bild des untersuchten Garns (5) darstellen.

8. Vorrichtung nach Anspruch 7 **dadurch gekennzeichnet, dass** das Messorgan (4) einen ersten Sensor zur Bestimmung der Masse oder des Durchmessers der Garnprobe (5) aufweist.

9. Vorrichtung nach Anspruch 7, **dadurch gekennzeichnet, dass** das Messorgan (4) einen zweiten und/oder einen dritten Sensor zur Bestimmung der Haarigkeit beziehungsweise der Struktur, insbesondere der Drehung, der Garnprobe (5) aufweist.

## Claims

1. Method for assessing the effects of parameters associated with the volume and/or the diameter and/or the surface of a yarn on a woven or knitted fabric produced from the respective yarn, by simulation of a fabric image, **characterised in that**
the yarn is moved in its longitudinal direction through a measuring member (4) which generates signals indicating the variations of an investigated parameter over the length of the test material, **in that** the signals are converted into grey values or colour values and these values are assigned to image spots which correspond to a position in the longitudinal direction of the yam and **in that** these image spots are arranged in series one after the other according to their position along the yarn, **in that** the said parameters are formed by the mass, diameter, hairiness and/or structure of the yam (5) and **in that** the conversion of the said parameters takes place according to the formula I = F(y), I designating the brightness or coulour step and y the respective parameter.

2. Method according to Claim 1, **characterised in that** the directly measured variations of the said parameters, the so-called graphs, are evaluated, and **in that** the values for the conversion are taken directly from the graphs, **in that** selected quantities relevant in textile terms are derived from the directly measured variations of the said parameters, and these derived quantities are used for the conversion, a yarn signal being simulated from these quantities relevant in textile terms in order to reproduce the image spots and **in that**, for the simulation, particular characteristics are emphasised by measures known from image processing, such as contrast accentuation, colouring and the like.

3. Method according to Claim 1, **characterised in that** the rare faults in the yarn (5), the so-called imperfections, are used for the conversion, and that these are represented in the image by means of the simulated yarn signal with an intensity and frequency corresponding to the intensity and frequency of the measured data.

**4.** Method according to Claim 1, **characterised in that** the wavelength spectrogram of the variations of a parameter is used for the conversion and examination of the wavelength spectrogram for so-called chimneys, that is to say values projecting appreciably above the ideal spectrogram curve, being carried out.

**5.** Method according to Claim 4, **characterised in that** the conversion takes place according to the formula

$$y = \sum_{i=1}^{n} \left[ a_i \sin \left( \frac{\Pi}{\lambda_i} x \right) \right]$$

$\lambda$ denoting the wavelength of a chimney, a its amplitude, i its index in the wavelength spectrogram and x the position of the chimney in the longitudinal direction of the yam sample (5), and y representing a reconstruction of the respective parameter.

**6.** Apparatus for assessing the effects of parameters associated with the volume and/or the diameter and/or the surface of a yam on a woven or knitted fabric produced from the respective yarn, by simulation of a fabric image, **characterised by** a measuring member (4) for determining signals which correspond to variations of a parameter over the length of the yarn, by a computer (2) for converting the signals into grey values or colour values, with means for assigning the grey values or colour values to image spots, by a video display unit (7) and/or printer (3) and by control means for reproducing the image spots on the video display unit and/or printer according ot the position in the longitudinal direction of the yarn, for the purpose of simulating a woven or knitted fabric produced from the examined yam, whereas the reproduced image spots represent a simulation of the examined yarn (5) which has been obtained by means of statistical examinations of quantities relevant in the textile terms of the yarn signal obtained during the test of the yarn sample.

**7.** Apparatus according to Claim 6, **characterised in that** the reproduced image spots represent a reconstructed image of the examined yarn.

**8.** Apparatus according to Claim 7, **characterised in that** the measuring member (4) has a first sensor for determining the mass or diameter of the yarn sample (5).

**9.** Apparatus according to Claim 7, **characterised in that** the measuring member (4) has a second and/or a third sensor respectively determining the hairiness or structure, in particular twist, of the yarn sample (5).

**Revendications**

**1.** Procédé pour apprécier les effets de paramètres liés au volume et/ou au diamètre et/ou à la surface d'un fil sur des tissus et tricots qui sont réalisés à partir du fil concerné, par une simulation de l'aspect du tissu, **caractérisé en ce que** le fil est amené à passer dans sa direction longitudinale à travers un organe de mesure (4) qui produit des signaux qui indiquent des oscillations d'un paramètre examiné sur la longueur du produit d'essai, que les signaux sont transformés en valeurs de gris ou en valeurs de couleur, et que celles-ci sont associées à des éléments d'image qui correspondent à une position dans la direction longitudinale du fil, et que ces éléments d'image, selon leur position, sont juxtaposés le long du fil,
**en ce que** les paramètres indiqués sont formés par la masse, le diamètre, la pilosité et/ou la structure du fil (5), et **en ce que** le calcul des paramètres indiqués a lieu selon la formule I = F (y), I désignant le degré de luminosité ou de couleur et y le paramètre concerné.

**2.** Procédé selon la revendication 1, **caractérisé en ce que** les oscillations mesurées directement des paramètres cités, les soi-disant diagrammes, sont évaluées, et que les valeurs pour la transformation sont prélevées directement des diagrammes,
**en ce que** sont dérivées, à partir des oscillations mesurées directement des paramètres cités, des grandeurs sélectionnées pertinentes du point de vue de la technique du textile, et que ces grandeurs dérivées sont utilisées pour la transformation, où pour la reproduction des éléments d'image à partir de ces grandeurs pertinentes du point de vue de la technique du textile, il est simulé un signal de fil et

**en ce que**, pour la simulation, on fait ressortir certaines caractéristiques par des mesures connues par le traitement de l'image, comme un renforcement du contraste, une coloration et analogues.

3. Procédé selon la revendication 1, **caractérisé en ce que** sont utilisés pour la transformation les défauts rares dans le fil (5), les soi-disant imperfections, et que ceux-ci sont représentés avec une intensité et fréquence correspondant à l'intensité et à la fréquence des données de mesure dans l'image avec le signal de fil simulé.

4. Procédé selon la revendication 1, **caractérisé en ce qu'**on utilise pour la transformation le spectrogramme des longueurs d'onde des oscillations d'un paramètre, où a lieu un examen du spectrogramme des longueurs d'onde, quant à des soi-disant cheminées, ce sont des valeurs dépassant d'une manière importante la courbe idéale du spectrogramme.

5. Procédé selon la revendication 4, **caractérisé en ce que** la transformation a lieu selon la formule

$$y = \sum_{i=1}^{n} \left[ a_i \cdot \sin(\frac{\pi}{\lambda_i} \cdot x) \right]$$

où, désigne la longueur d'onde d'une cheminée, a l'amplitude de celle-ci, et i l'indice de celle-ci dans le spectrogramme des longueurs d'onde, et x la position de la cheminée dans la direction longitudinale de l'échantillon de fil (5), et y représente une reconstruction du paramètre concerné.

6. Dispositif pour apprécier les effets de paramètres liés au volume et/ou au diamètre et/ou à la surface d'un fil sur des tissus et tricots, qui sont fabriqués à partir du fil concerné, par la simulation de l'aspect du tissu, **caractérisé par** un organe de mesure (4) pour déterminer des signaux qui correspondent à des oscillations d'un paramètre sur la longueur du fil, par un calculateur (2) pour transformer les signaux en valeurs de gris ou en valeurs de couleur, avec des moyens pour associer les valeurs de gris ou les valeurs de couleur à des éléments d'image, par un écran (7) et/ou une imprimante (3) et par des moyens de commande pour reproduire les éléments d'image sur l'écran et/ou l'imprimante, selon la position dans la direction longitudinale du fil, en vue de la simulation d'un tissu ou tricot, fabriqué à partir du fil examiné, où les éléments d'image reproduits représentent une simulation du fil examiné (5), qui ont été obtenus à l'aide de contrôles statistiques de grandeurs pertinentes, du point de vue de la technique du textile, des signaux de fil obtenus lors de l'examen de l'échantillon de fil.

7. Dispositif selon la revendication 6, **caractérisé en ce que** les éléments d'image reproduits représentent une image reconstruite du fil examiné (5).

8. Dispositif selon la revendication 7, **caractérisé en ce que** l'organe de mesure (4) présente un premier capteur pour déterminer la masse ou le diamètre de l'échantillon de fil (5).

9. Dispositif selon la revendication 7, **caractérisé en ce que** l'organe de mesure (4) présente un deuxième et/ou un troisième capteur pour déterminer la pilosité, respectivement la structure, notamment la torsion, de l'échantillon de fil (5).

FIG. 1

M

| 0 | 50 | 100 | 150m |

FIG. 2

Probe 2

FIG. 3

Probe 1

| 0.01 | 0.1 | 1 | 10 | 100 | 1000m |

EP 0 578 975 B2

FIG. 4a

10

FIG. 4b